# EUROPEAN PATENT SPECIFICATION

(11) **EP 0 955 358 B1**
(45) Date of publication and mention of the grant of the patent: **12.09.2007**
(21) Application number: 99104563.4
(22) Date of filing: 08.03.1999
(51) Int. Cl.: C12N 1/21, C12P 17/04, C12P 19/02, C12N 9/04

(54) **Genetically engineered L-sorbose reductase-deficient mutants**
Genetisch veränderte, L-Sorbose Reduktase defiziente Mutanten
Mutants génétiquement modifiés ayant une activité L-sorbose reductase déficiente

(30) Priority: 13.03.1998 EP 98104546
(43) Date of publication of application: 10.11.1999
(73) Proprietor: DSM IP Assets B.V., 6411 TE Heerlen (NL)
(72) Inventor: Hoshino, Tatsuo, Kamakura-shi, Kanagawa-ken (JP); Kon, Takahide, Yokohama-shi, Kanagawa-ken (JP); Shinjoh, Masako, Kamakura-shi, Kanagawa-ken (JP); Tazoe, Masaaki, Yokohama-shi, Kanagawa-ken (JP)
(74) Representative: Seibel-Thomsen, Nadja

(56) References cited:
- EP-A- 0 233 050
- EP-A- 0 728 840

### SEQUENCE LISTING

<110> F. Hoffmann-La Roche AG
<120> Genetically engineered L-sorbose reductase-deficient mutants
<130> Case 20079
<140> 99.104563.4<141> 1999-03-08
<150> 98.104546.1
   <151> 1998-03-13
<160> 4
<170> PADAT Sequenzmodul, Version 1.0
<210> 1
   <211> 1458
   <212> DNA
   <213> Gluconobacter suboxydans
<220>
   <223>
<400> 1
   atgatcacgc acgaaaccct caagtctctt cccgccggtg tgcaggctcc gccctatgac   60
   atcaatggga tcaaaccggg gatcgtgcat tttggcgtgg gaaacttctt ccgggcccat  120
   gaggctttct acgttgaaca gatcctcaag gacgatccga actggggaat catcggcgtt  180
   ggtctgacgg gtagcgacag gtcaaagaag aaggccgagg aattcaagaa gcaggactgc  240
   ctcttttccc tgaccgaaac ggctccgtcc ggcaagagca cggttcgtgt tatgggcgcg  300
   ctgagggatt accttttggc tcctgccgat ccggaagccg tgctgaagca tctcgctgac  360
   ccgggaatcc gtatcgtttc catgacaatc acggaaggcg gttacaacat taacgagacg  420
   acaggtgagt tcgatcttga gaacaaggcg gttcagcagg atctgaagac acccgaaacg  480
   ccgtccacaa tctttggata tgttgtggaa ggactgcgcc gccgccgtga cgcaggtggc  540
   aaggccttca cgatcatgtc ctgcgataat ctgcggcata acggtaatgt cgcccgcaag  600
   gcatttctgg gatacgcgaa ggcccgtgat ccggaactgg ccaagtggat tgaagagaac  660
   gcgacgttcc caaatggcat ggttgatcgc atcacgccga ccgtttctgc tgacattgcg  720
   aagaagctca acgaagccag tggcctgcac gacgacctgc cgctcgttgc agaagacttt  780
   catcagtggg tgctggaaga cagctttgct gatggccggc ctgcgctgga aaaggccgga  840
   gtgcagttcg ttggggatgt gacggactac gagcatgtaa aaatccgcat gctgaatgct  900
   ggtcacatca tgctctgctt cccggctgtt ctggcaggat ttgaaaatgt cgatcatgcc  960
   cttgctgatc ccgatctacg gcgtatcctc gagaacttcc tgaacaaaga cgtcatcccg 1020
   accctgaagg caccgccggg catgacgctg gaaggctatc gggacagcgt gatcagccgt 1080
   ttctcgaatc cggccatggc ggatcagaca ttgcgtattt ccggggacgg gagctcgaag 1140
   atccaggtct tctggacgga aacggtccgc aaggcttttg agggcaagcg cgatctgtcc 1200
   cgcattgctt ttggtatggc atcctacctg gaaatgctgc gcggtaagga tgaaacgggt 1260
   ggcacctacg agccattcga gccgactttt ggtgacaacc ataagactct ggccaaggct 1320
   gatgattttg agagcgcgct caagctgcca gcgttcgatg cctggcgcga tctggagacg 1380
   tccgggctga acaacaaggt tgtggagctt cgcaagatta tccgcgagaa gggcgtcaag 1440
   gctgcccttc cggcctga                                               1458
<210> 2
   <211> 485
   <212> PRT
   <213> Gluconobacter suboxydans
<400> 2
   Met Ile Thr His Glu Thr Leu Lys Ser Leu Pro Ala Gly Val Gln
    1               5                  10                  15
   Ala Pro Pro Tyr Asp Ile Asn Gly Ile Lys Pro Gly Ile Val His
                   20                  25                  30
   Phe Gly Val Gly Asn Phe Phe Arg Ala His Glu Ala Phe Tyr Val
                   35                  40                  45
   Glu Gln Ile Leu Lys Asp Asp Pro Asn Trp Gly Ile Ile Gly Val
                   50                  55                  60
   Gly Leu Thr Gly Ser Asp Arg Ser Lys Lys Lys Ala Glu Glu Phe
                   65                  70                  75
   Lys Lys Gln Asp Cys Leu Phe Ser Leu Thr Glu Thr Ala Pro Ser
                   80                  85                  90
   Gly Lys Ser Thr Val Arg Val Met Gly Ala Leu Arg Asp Tyr Leu
                   95                  100                 105
   Leu Ala Pro Ala Asp Pro Glu Ala Val Leu Lys His Leu Ala Asp
                   110                 115                 120
   Pro Gly Ile Arg Ile Val Ser Met Thr Ile Thr Glu Gly Gly Tyr
                   125                 130                 135
   Asn Ile Asn Glu Thr Thr Gly Glu Phe Asp Leu Glu Asn Lys Ala
                   140                 145                 150
   Val Gln Gln Asp Leu Lys Thr Pro Glu Thr Pro Ser Thr Ile Phe
                   155                 160                 165
   Gly Tyr Val Val Glu Gly Leu Arg Arg Arg Arg Asp Ala Gly Gly
                   170                 175                 180
   Lys Ala Phe Thr Ile Met Ser Cys Asp Asn Leu Arg His Asn Gly
                   185                 190                 195
   Asn Val Ala Arg Lys Ala Phe Leu Gly Tyr Ala Lys Ala Arg Asp
                   200                 205                 210
   Pro Glu Leu Ala Lys Trp Ile Glu Glu Asn Ala Thr Phe Pro Asn
                   215                 220                 225
   Gly Met Val Asp Arg Ile Thr Pro Thr Val Ser Ala Asp Ile Ala
   230 235 240
   Lys Lys Leu Asn Glu Ala Ser Gly Leu His Asp Asp Leu Pro Leu
   245 250 255
   Val Ala Glu Asp Phe His Gln Trp Val Leu Glu Asp Ser Phe Ala
   260 265 270
   Asp Gly Arg Pro Ala Leu Glu Lys Ala Gly Val Gln Phe Val Gly
   275 280 285
   Asp Val Thr Asp Tyr Glu His Val Lys Ile Arg Met Leu Asn Ala
   290 295 300
   Gly His Ile Met Leu Cys Phe Pro Ala Val Leu Ala Gly Phe Glu
   305 310 315
   Asn Val Asp His Ala Leu Ala Asp Pro Asp Leu Arg Arg Ile Leu
   320 325 330
   Glu Asn Phe Leu Asn Lys Asp Val Ile Pro Thr Leu Lys Ala Pro
   335 340 345
   Pro Gly Met Thr Leu Glu Gly Tyr Arg Asp Ser Val Ile Ser Arg
   350 355 360
   Phe Ser Asn Pro Ala Met Ala Asp Gln Thr Leu Arg Ile Ser Gly
   365 370 375
   Asp Gly Ser Ser Lys Ile Gln Val Phe Trp Thr Glu Thr Val Arg
   380 385 390
   Lys Ala Phe Glu Gly Lys Arg Asp Leu Ser Arg Ile Ala Phe Gly
   395 400 405
   Met Ala Ser Tyr Leu Glu Met Leu Arg Gly Lys Asp Glu Thr Gly
   410 415 420
   Gly Thr Tyr Glu Pro Phe Glu Pro Thr Phe Gly Asp Asn His Lys
   425 430 435
   Thr Leu Ala Lys Ala Asp Asp Phe Glu Ser Ala Leu Lys Leu Pro
   440 445 450
   Ala Phe Asp Ala Trp Arg Asp Leu Glu Thr Ser Gly Leu Asn Asn
   455 460 465
   Lys Val Val Glu Leu Arg Lys Ile Ile Arg Glu Lys Gly Val Lys
   470 475 480
   Ala Ala Leu Pro Ala
   485
<210> 3
   <211> 8
   <212> PRT
   <213> Gluconobacter melanogenus
<400> 3
   Met Thr Ile Thr Glu Gly Gly Tyr
    1              5           8
<210> 4
   <211> 8
   <212> PRT
   <213> Gluconobacter melanogenus
<400> 4
   Phe Pro Asn Gly Met Val Asp Arg
    1              5           8

## Description

The present invention relates to novel genetically engineered L-sorbose reductase-deficient mutants of a microorganism belonging to the genus *Gluconobacter* or *Acetobacter* which are of advantage in the use for the production of L-sorbose by fermentation as well as in improvement of vitamin C production process.

The production of vitamin C has been conducted by Reichstein method which involves a fermentation process for the conversion from D-sorbitol to L-sorbose by a microorganism belonging to the genus *Gluconobacter* or *Acetobacter*, as a sole biological step. The said conversion to L-sorbose is one of the key steps for the efficacy of vitamin C production. It was, however, observed that the product, L-sorbose, was consumed after the consumption of the substrate, D-sorbitol, during the oxidative fermentation with the said microorganism. This phenomenon was understood that L-sorbose was reduced by NADPH-linked L-sorbose reductase (hereinafter occasionally referred to as SR) present in the cytosol (See: Sugisawa et al., Agric. Biol. Chem. 55: 2043-2049, 1991). It was reported that, in *Gluconobacter*, D-sorbitol was converted to D-fructose which could be incorporated into the pentose pathway and further metabolized to CO₂ (Shinjoh et al., Agric. Biol. Chem. 54: 2257-2263, 1990). Such pathways consuming the product as well as the substrate might have caused less productivity of vitamin C ultimately.

An improvement of L-sorbose production was reported by Nogami et al. in Japanese Patent Application Kokai No. 51054/1995. They subjected the micro-organisms of *Gluconobacter oxydans* and *Gluconobacter suboxydans* to conventional chemical mutagenesis and isolated the mutant strains whose ability of utilizing D-sorbitol as a sole assimilable carbon source was reduced. By applying such mutant to the fermentation for L-sorbose production, they observed more than 2-3 % improvement of the productivity in comparison with the productivity of the parent strain.

One of the disadvantages which is often observed in mutant strains produced by the conventional mutagenesis is back mutation which nullifies the improved characteristics of the mutant strains during the course of fermentation or subculture of the mutant, which would result in decreased productivity of vitamin C ultimately. Therefore a stably blocked mutant of L-sorbose reductase is desired.

In one aspect, the present invention provides a novel genetically engineered microorganism derived from a microorganism belonging to the genus *Gluconobacter* or *Acetobacter* which is characterized in that the biological activity thereof for reducing L-sorbose is substantially nullified by means of genetic engineering. And it has the said biological activity less than 10% of that of the original microorganism; the said activity is e.g. 0.07 to 0.02 units/mg protein or less according to the definition of the activity described below (e.g. in Example 1 (iii)). The gene of the genetically engineered microorganism of the present invention must carry a stably integrated mutation, preferably at least one mutation with the aid of disruption, addition, insertion, deletion and/or substitution of nucleotide(s) within the region required for the formation of active L-sorbose reductase in cells of the microorganism.

In one preferable embodiment of the genetically engineered microorganism of the present invention, the said mutation may be caused by the gene disruption within the region required for the formation of active L-sorbose reductase. Such disruption may contain at least one interfering DNA fragment selected from the group consisting of a transposon, an antibiotics resistant gene cassette and any DNA sequences which prevent the host microorganism from the formation of active L-sorbose reductase.

In another embodiment of the present invention, the said mutation may be produced by mutagenesis with the aid of site-directed mutagenesis. Such mutagenesis can be effected in the region required for the formation of active L-sorbose reductase, which region may include a structural gene of L-sorbose reductase and expression control sequences, such as promoter, operator, terminator, DNA encoding repressor, activator and the like.

Another aspect of the present invention provides the use of an L-sorbose reductase gene of a microorganism belonging to the genus *Gluconobacter* or *Acetobacter* in producing the genetically engineered microorganism as described above, in which the said gene is characterized in that it encodes the amino acid sequence of L-sorbose reductase described in SEQ ID NO: 2 or its functional equivalents containing insertion, deletion, addition and/or substitution of one or more amino acid(s) in said SEQ ID NO: 2.

A further aspect of the present invention provides an efficient method for producing L-sorbose by the fermentation of a microorganism in an appropriate medium, which comprises use of the genetically engineered microorganism of the present invention described above. In connection to this L-sorbose production method, the present invention also provides an efficient vitamin C production process containing a fermentation step for the production of L-sorbose which is characterized in that the said fermentation is carried out by using the genetically engineered microorganism of the present invention as described above.

The present invention provides a novel genetically engineered microorganism derived from a microorganism belonging to the genus *Gluconobacter* or *Acetobacter* which is characterized in that the biological activity thereof for reducing L-sorbose is substantially nullified by means of genetic engineering.

The said genetically engineered microorganism may be a microorganism belonging to genus *Gluconobacter* or *Acetobacter*, which includes *Gluconobacter albidus*, *Gluconobacter capsulatus*, *Gluconobacter cerinus*, *Gluconobacter dioxyacetonicus, Gluconobacter gluconicus*, *Gluconobacter industrius*, *Gluconobacter melanogenus* (IFO 3293 and FERM P-8386 [National Institute of Bioscience and Human-Technology, Japan]), *Gluconobacter nonoxygluconicus, Gluconobacter oxydans, Gluconobacter oxydans subsp. sphaericus, Gluconobacter roseus, Gluconobacter rubiginosus, Gluconobacter suboxydans* (IFO 3291), *Acetobacter xylinum* [commercially available from the Institute of Fermentation, Osaka, Japan (IFO) as IFO 3288], *Acetobacter pasteurianus, Acetobacter aceti*, *Acetobacter hansenii* and *Acetobacter liquefaciens* (IFO 12388; ATCC 14835). For strain information see also European Patent Application Publ. No. (EPA) 213591 and 518136. *Gluconobacter suboxydans* IFO 3291 has been deposited in the form of a mixture with *Gluconobacter oxydans* DSM4025 as FERM BP-3813 and *Gluconobacter melanogenus* IFO 3293 as FERM BP-8256. Further details can be taken from EP 518136. For *Gluconobacter suboxydans* (IFO 3291) and *Gluconobacter melanogenus* (IFO 3293) see also EP 728 840 (US patent application No. 08/606 807).

For the purpose of nullifying the biological activity of the said microorganism in reducing L-sorbose, the present invention involves genetic engineering to target the region of the gene of the microorganism required for the formation of the active L-sorbose reductase. The typical methodologies for this purpose are known as gene disruption with transposon or selection marker gene cassette and site-directed mutagenesis. As it is described below, gene disruption method is useful to identify the target gene of the microorganism as well as to block the gene function. Once the above mentioned target region of the gene has been identified, conventional mutagenesis can also be applicable by the treatment of the target DNA fragment with e.g. a chemical mutagen, ultra violet irradiation and the like.

Gene disruption may be carried out by the introduction of an interfering DNA fragment into chromosomal DNA of the microorganism with the aid of transposon mutagenesis, introduction of a gene cassette carrying a selection marker, such as antibiotics resistant gene, DNA sequences which nullify the formation of active L-sorbose reductase.

### (a) Transposon mutagenesis:

Transposon mutagenesis is known as a potent tool for genetic analysis (P. Gerhardt et al., "Methods for General and Molecular Bacteriology" Chapter 17, Transposon Mutagenesis; American Society for Microbiology). This method utilizes a transposable elements which are distinct DNA segments having the unique capacity to move (transpose) to new sites within the genome of the host organisms. The transposition process is independent of the classical homologous recombination system of the organism. The insertion of a transposable element into a new genomic site does not require extensive DNA homology between the ends of the element and its target site. Transposable elements have been found in a wide variety of prokaryotic and eukaryotic organisms, where they can cause null mutations, chromosome rearrangements, and novel patterns of gene expression on insertion in the coding region or regulatory sequences of resident genes and operons.

Prokaryotic transposable elements can be roughly divided into three different classes. Class I consists of simple elements such as insertion sequences (IS elements), which are approximately 800 to 1,500 bp in length. IS elements normally consist of a gene encoding an enzyme required for transposition (i.e. transposase), flanked by terminally repeated DNA sequences which serve as substrate for the transposase. IS elements were initially identified in the lactose and galactose utilization operons of enteric bacteria, where the elements were found to cause often unstable, polar mutation on insertion.

Class II consists of composite transposable elements. The members of this class are also referred to as transposons or Tn elements. Transposons in prokaryotes have been identified as a class of complex transposable elements, often containing simple IS elements (or parts thereof) as direct or inverted repeats at their termini, behaving formally like IS elements but carrying additional genes unrelated to transposition functions, such as antibiotic resistance, heavy-metal resistance or pathogenicity determinant genes. The insertion of a transposon into a particular genetic locus or replicon (phage) is designated by using a double colon, e.g. *lacZ*::Tn5 or 1::Tn5.

Class III includes "transposable" bacteriophages, such as Mu and its relatives. Phage Mu is both a virus and a transposon. It is known that it can integrate at multiple sites in the host chromosome, thereby frequently causing mutations.

The transposon mutagenesis utilizing the above transposable elements is known to possess the following characteristics:
(i) Such mutation generally leads to inactivation of the gene, and the resulting null mutation is relatively stable.
(ii) Transposons introduce new genetic and physical markers into the target locus, such as antibiotic resistance genes, new restriction endonuclease cleavage sites, and unique DNA sequences which can be identified by genetic means, DNA-DNA hybridization, or electron microscopic heteroduplex analysis. The genetic markers are useful for mapping the mutated loci as well as screening the mutants.
(iii) Transposons can generate a variety of genomic rearrangements, such as deletions, inversions, translocations, or duplications, and can be used to introduce specific genes into the target bacteria.

A variety of transposons are known in the art, such as Tn3, Tn5, Tn7, Tn9, Tn10, phage Mu and the like. Among them, Tn5 is known to have almost no insertion specificity, and its size is relatively small. Tn5 is also one of the most frequently used transposable elements which is readily derived from the sources, such as pfd-Tn5 [American Type Culture Collection, USA (ATCC) ATCC 77330] or pCHR81 (ATCC 37535). For the purpose of use in the random mutagenesis in the practice of the present invention, Tn5 is preferred. A variety of Tn5 derivatives, designated Mini-Tn5s, which consists of 19 bp of the Tn5 inverted repeats required for transposition coupled to antibiotic resistance or other selectable marker genes are also useful for the present invention. Such Mini-Tn5s are inserted into a suicide vector, in addition to the Tn5 transposase (*tnp*), to construct an efficient suicide Tn5 mutagenesis system. Further information how to work with Tn5 transposons can be taken from the following references, P. Gerhardt et al., "Methods for General and Molecular Bacteriology" Chapter 17, Transposon Mutagenesis; American Society for Microbiology, 1994; K.N. Timmis et al., Mini-Tn5 transposon derivatives for insertion mutagenesis, promoter probing, and chromosomal insertion of cloned DNA in Gram-negative Eubacteria, J. Bacteriology, 172: 6568-6572, 1990.

Information describing how Tn5 can be derived from pfd-Tn5 see explanation of pfd-Tn5 (ATCC 77330) obtainable from the ATCC Home Page on Internet, [http://www.atcc.org/catalogs/recomb.html]. Accordingly the suicide plasmid pfd-Tn5 can be introduced into *E. coli* as well as other Gram negative bacterium by electroporation (see the reference for recommended conditions). The plasmid itself can be used as a Tn5 donor. In addition, one can construct a suicide Tn5 vector by introducingpfd-Tn5 plasmid into the recipient *E. coli* with any plasmid which one wants to use, selecting transformants showing Km^{r} and resistance of the target plasmid, isolating plasmids from the transformants, transforming *E. coli* with the isolated plasmids, and selecting for Km and plasmid-marker-resistant transformants to obtain *E. coli* strain carrying the target palsmid with Tn5. Concept of this protocol is also available in "region-directed Tn5 mutagenesis" in P. Gerhardt et al. (mentioned above).

Random mutagenesis with transposon involves the introduction of a transposon into a target bacterial cell via transformation, transduction, conjugal mating or electroporation by using suicide plasmid or phage vectors. The resulting mutants may be screened with the aid of the marker carried by the transposon. Transposition of the transposon into the genome of the recipient bacterium can be detected after the vector used has been lost by segregation.

For the introduction of transposons into a microorganisms of the genus *Gluconobacter* or *Acetobacter,* so-called suicide vectors including a derivative of phage P1 and narrow-host-range plasmids are commonly used. The phage P1 vectors and the plasmid vectors can be transferred by infection and by transformation, conjugal mating or electroporation, respectively, into the recipient cells, wherein these vectors preferably lack the appropriate origins of recipients. The choice of suicide vector and transposon to be used depends on criteria including phage sensitivity, intrinsic antibiotic resistance of the recipient cell, the availability of a gene transfer system including transformation, conjugal transfer, electroporation, or infection to introduce transposon-carrying vector into *E. coli*.

One of the preferable vectors for use in the present invention is phage P1 (ATCC25404) which injects its DNA into a microorganism belonging to the genus *Gluconobacter* or *Acetobacter*, however, this DNA will be unable to replicate and will be lost by segregation. Such P1 phage carrying Tn5 (P1::Tn5) can be used in the form of phage lysate which may be prepared by lysing *E. coli* carrying P1::Tn5 in accordance with known procedures (see: e.g. Methods for General and Molecular Bacteriology" Chapter 17, Transposon Mutagenesis; American Society for Microbiology or US patent 5082785, 1992).

The other preferable suicide vectors which can be used in the present invention are plasmid suicide vectors, which may be based on replicon derived from plasmid RP4, or its relative RK2 (ATCC37125), carrying the same broad-host-range conjugal transfer of mobilization functions and sites but a narrow-host-range origin of replication. These vectors can be mobilized at a high frequency from *E. coli* to a microorganism belonging to the genus *Gluconobacter* or *Acetobacter* but cannot be stably maintained in the recipient cells. These vectors contain, in addition to Tn5, the IncP-type mobilization (*mob; oriT*) site and are based on commonly used *E. coli* cloning vectors, such as pACYC177 (ATCC37031), pACYC184 (ATCC37033), and pBR325 [Bolivar F., 1978, Gene 4: 121-136; a derivative of pBR322 (ATCC31344)], all of which cannot replicate in nonenteric bacteria (pSUPseries, Simon R. et al., 1983, Bio/Technology 1: 784-791). pSUP-type plasmids can be mobilized in bi-parental mating experiments by providing the transfer function in trans from a chromosomally integrated copy of the IncP plasmid RP4 in the donor strain itself (e. g., strain S17-1) or in tri-parental mating experiments by providing the transfer functions from plasmid pRK2013 (ATCC37159) harbored by a nondonor, nonrecipient helper strain of *E. coli*.

The recipient cell which received the transposon can be selected by the marker carried by the element, e.g. resistance to particular antibiotics. When Tn5 is used as a transposon, the marker of Km^{r} or Nm^{r} can be usually used. Besides Km^{r} or Nm^{r} markers, genetic markers of Tc^{r}, Gm^{r}, Sp^{r}, Ap^{r}, Cm^{r} and the like can be used as the alternative marker genes. The other transposons carrying readily visualized gene products such as those encoded by *lacZ*, *luxAB* or *phoA* can also be used. These Tn5 derivatives are especially useful if the target bacterial strain has an intrinsic resistance to the antibiotics normally used to select for Tn5 (kanamycin, neomycin, bleomycin and streptomycin) or if a secondary mutagenesis of a strain already harboring a Tn5 derivative is to be carried out (P. Gerhardt et al., "Methods for General and Molecular Bacteriology" Chapter 17, Transposon Mutagenesis; American Society for Microbiology).

### (b) Region-directed mutagenesis

A powerful extension of the Tn5 mutagenesis protocol is the use of gene replacement techniques to substitute the wild-type gene in the original bacterial strain with its well-characterized Tn5-mutated analog carried by a plasmid in *E. coli*. When a wild-type locus has been cloned, region-directed mutagenesis with Tn5 or its derivatives or gene cassettes carrying selection markers can be used efficiently to inactivate the target genes and operons carried by the cloned region. For this purpose, Tn5 itself or its derivatives mentioned above or any gene cassettes with selection markers, e. g., Km^{r} gene cassette carried by pUC4K (Pharmacia, Uppsala, Sweden) can be used. This approach is very efficient when one likes to inactivate the gene of interest, e. g., the gene of a mutant strain developed from the parent strain from which the wild-type gene was once cloned. The only limitation in these types of replacement experiments is the length of homologous sequences needed for the double-crossover recombination event; 0.5 - 5 kb long homologous sequences at both ends are preferably required.
The preferable vectors for the region-directed mutagenesis are the same as those useful for transposon mutagenesis; above mentioned suicide phage and plasmid vectors.

### (c) Site-directed mutagenesis

When a wild-type gene is cloned and its nucleotide sequence is determined, site-directed mutagenesis can also be used to inactivate the target genes. Oligonucleotide primers including addition and deletion of nucleotides for frame-shift, or substitution of nucleotides for introduction of stop codons or different codons are used for mutating genes of interest. The mutagenesis with the primers including mutation(s) can be conducted usually in *E. coli* with any commercial site-directed mutagenesis kits. This-type mutagenesis is useful if Tn5- or region directed-cassette-mutagenesis causes polar effect affecting gene expression of downstream or upstream.
The preferable vectors for introducing the mutated gene into the target microorganism are the same as those useful for transposon mutagenesis. In this case, aimed mutant can be isolated by biological assay, e. g., enzymological or immunological screening to detect deficiency of the target gene product. Instead, the mutated gene can be tagged with selection marker gene described above at the site not affecting gene expression of downstream or upstream to facilitate the selection of the gene replacement. One can obtain the target gene disruptant more easily by combination of biological assay with marker-selection.

The L-sorbose reductase-deficient mutant can be selected generally as follows: 3,000 to 10,000 transposon mutants are subjected to the product assay with L-sorbose as the substrate to select the mutant which does not convert L-sorbose to D-sorbitol. The first screening can be done preferably in microtiter plates with the reaction mixture containing L-sorbose. The formation of the product, D-sorbitol, is firstly detected by TLC with appropriate developing solvent; candidates forming undetectable amount of D-sorbitol are selected.
Then, the candidate mutants are subjected to assay of L-sorbose reductase activity as exemplified in the Example 1 of the present invention to confirm L-sorbose reductase-deficiency.
For confirming that the deficient mutant really carries transposon, colony- or Southern-hybridization is usually conducted with labeled-DNA fragment containing the transposon used as the probe by the standard methods (Molecular cloning, a laboratory manual second edition, Maniatis T., et al., 1989).
Such a mutant was isolated as described in Example 1 of the present invention. The transposon mutant is useful for further identifying the target L-sorbose reductase gene and determining its nucleotide sequence of the region tagged with the transposon.

The DNA fragment inserted by a transposon can be cloned into any *E. coli* cloning vectors, preferably pUC18, pUC19, pBluescript II (Stratagene Cloning Systems, CA, USA) and their relatives, by selecting transformants showing both phenotypes of selection markers of the vector and the transposon. The nucleotide sequences adjacent to the transposon are able to be determined by e.g., a chain termination method (Sanger F. S., et al., Proc. Natl. Acad. Sci. USA 74:5463-5467,1977). The resulting nucleotide sequences may be partial sequences whose reading frame might be difficult to be determined.
Once the nucleotide sequences were determined, they can be subjected to homology search performed with nucleotide and/or protein sequence data bases by using genetic analysis program, e. g., BLASTP search (Lipman et al., J. Mol. Biol. 215: 403-410, 1990). If any homologous sequences are found, their amino acid sequences can be aligned to find any consensus sequences which are conserved between the homologous proteins. According to the consensus sequences, oligonucleotide primers can be synthesized and used to amplify the partial DNA of the target gene by polymerase chain reaction (PCR). Besides the consensus sequences, any amino acid sequences, which are determined after adjusting the reading frame by the alignment of homologous proteins, can be used for designing the PCR primers.

The resulting PCR-born partial gene can be used as a probe to obtain the whole target gene through Southern- and colony-hybridization. Southern-hybridization reveals size of the DNA fragment containing the target gene and one can construct a mini-gene library containing the DNA fragments with the aimed size. The mini-library can then be screened with the partial gene as the probe by colony-hybridization to obtain the whole target gene. Then complete nucleotide sequence of the target gene can be determined to identify its open reading frame.

The region inserted by a transposon may be a regulatory gene controlling the expression of the structure gene of L-sorbose reductase; the regulatory gene can also be the target for disrupting L-sorbose reductase gene.

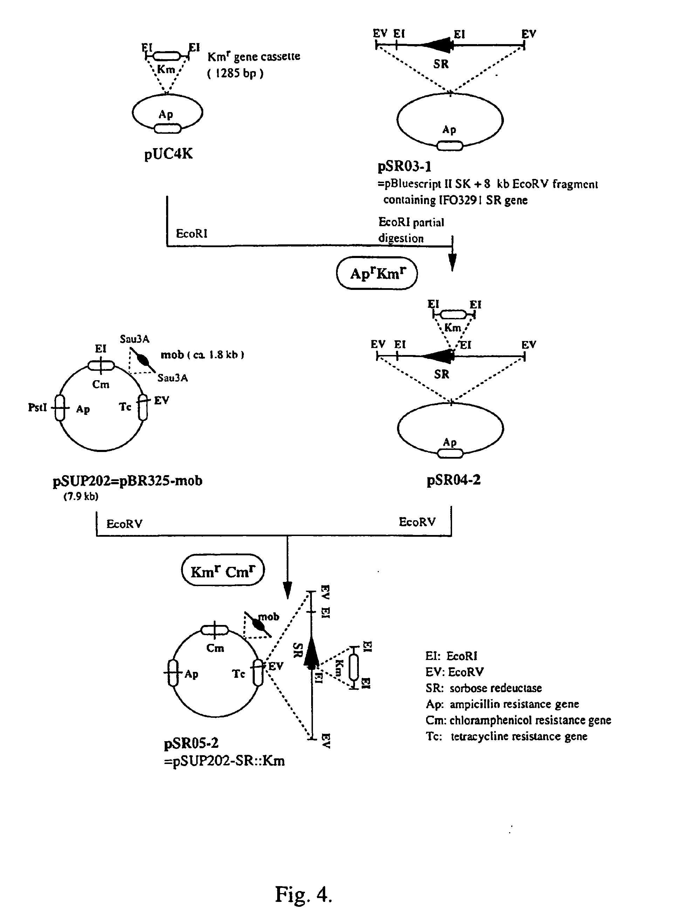

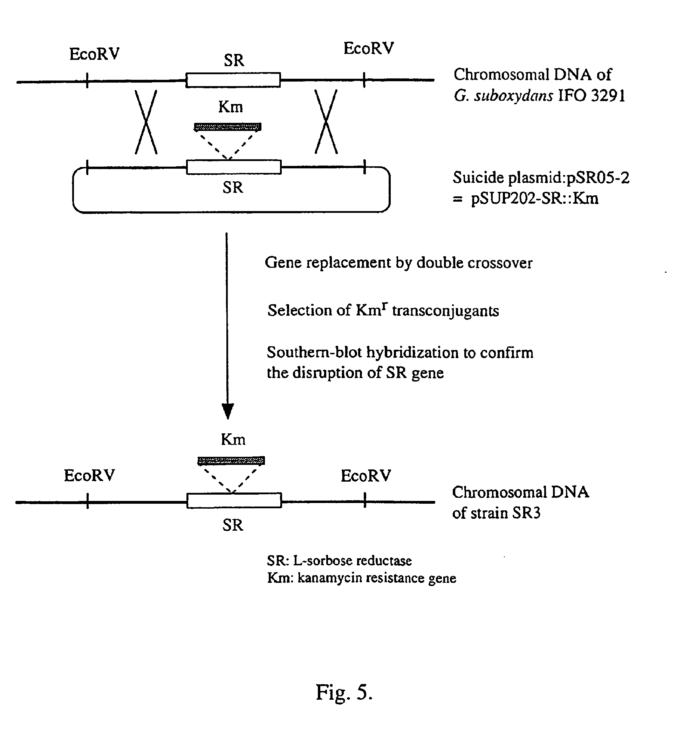

Cloned DNA fragment containing the partial or whole L-sorbose reductase gene of the target microorganism can be used for disrupting the L-sorbose reductase gene of the target microorganism. A schematic procedure and mechanism for the disruption are illustrated in Fig. 4 and 5, respectively. The DNA fragment is first cloned into *E. coli* vector such as pBluescript II SK. Then a gene cassette carrying a selection marker such as Km^{r} gene is inserted into the target L-sorbose reductase gene not to form active L-sorbose reductase. The resulting DNA fragment with disrupted L-sorbose reductase gene is recloned on a suicide vector such as pSUP202. The suicide plasmid carrying disrupted gene can be introduced into the recipient microorganism by any gene transfer methods including conjugal mating as described above. Selection of the target mutant generated by double crossover recombination event can be done by isolating colonies expressing the selection marker gene (e.g., Km^{r}) and characterizing its chromosomal DNA by Southern-blot hybridization. The L-sorbose reductase deficiency of the candidate mutant is confirmed not to show detectable enzyme activity of L-sorbose reductase.

Such a mutant was isolated as described in Example 5 of the present invention as the strain SR3. Non-assimilation of L-sorbose can be examined with any media containing 1-500 g/L of D-sorbitol for L-sorbose fermentation by chasing the concentration of L-sorbose once converted from D-sorbitol in the fermentation broth. Instead, L-sorbose-containing medium can also be used for confirming non-assimilation of L-sorbose under fermentation conditions.

The mutants provided in the present invention may be cultured in an aqueous medium supplemented with appropriate nutrients under aerobic conditions. The cultivation may be conducted at pH between about 3.0 and 9.0, preferably between about 5.0 and 8.0. While the cultivation period varies depending upon pH, temperature and nutrient medium used, usually 1 to 6 days will bring about favorable results. A preferred temperature range for carrying out the cultivation is from about 13°C to 45°C preferably from about 18°C to 42°C.

It is usually required that the culture medium contains such nutrients as assimilable carbon sources, digestible nitrogen sources and inorganic substances, vitamins, trace elements and the other growth promoting factors. As assimilable carbon sources, glycerol, D-glucose, D-mannitol, D-fructose, D-arabitol, D-sorbitol, L-sorbose, and the like can be used.

Various organic or inorganic substances may also be used as nitrogen sources, such as yeast extract, meat extract, peptone, casein, corn steep liquor, urea, amino acids, nitrates, ammonium salts and the like. As inorganic substances, magnesium sulfate, potassium phosphate, ferrous and ferric chlorides, calcium carbonate and the like may be used.

The L-sorbose reductase-deficient mutant of the present invention can be applied to the fermentative oxidation of D-sorbitol and is expected to improve the yield of vitamin C by increasing L-sorbose usable for the step of condensation reaction of L-sorbose to produce diacetone-L-sorbose. As it is apparent for those skilled in the art, the L-sorbose reductase-deficient mutant of the present invention may be applied to any processes for vitamin C production which include L-sorbose as a reaction intermediate.

The present invention is further illustrated with Examples described below by referring to the attached drawings which show as described below:
Figure 1 illustrates the nucleotide sequences upstream and downstream of Tn5-inserted region of the chromosomal DNA of L-sorbose reductase-deficient mutant, 26-9A derived from *G. melanogenus* IFO 3293.
Figure 2 illustrates oligonucleotide primers used for PCR cloning of SR gene from *G. suboxydans* IFO 3291.
Figure 3 illustrates Restriction map of 8.0 kb EcoRV fragment carrying L-sorbose reductase gene of *G. suboxydans* IFO 3291 (upper) and its enlarged region near the L-sorbose reductase gene (lower) showing ORFs found.
Figure 4 is a scheme for the construction of a suicide plasmid for disruption of L-sorbose reductase gene in *G. suboxydans* IFO 3291.
Figure 5 illustrates a schematic mechanism for the disruption of L-sorbose reductase gene of *G. suboxydans* IFO 3291.
Figure 6 shows the graphs illustrating the fermentation profiles of strain SR3 and *G. suboxydans* IFO 3291 in 8% sorbitol-No. 5 medium.
Figure 7 shows the graphs illustrating the fermentation profiles of strain SR3 and *G. suboxydans* IFO 3291 in 2% sorbitol-SCM medium.

### Example 1. Isolation of L-sorbose reductase-deficient Tn5 mutant from G. melanogenus IFO 3293 derivative

### (i) Tn5 mutagenesis.

Transposon mutagenesis was carried out (Manning R. F. et al., USP5082785) to construct L-sorbose reductase deficient strain from 2-keto-L-gulonic acid-producing L42-9 strain obtained from *G. melanogenus* IFO 3293 by means of multi-step mutations with chemical mutagens including NTG and ICR170, ultraviolet irradiation etc. *E. coli* W3110 carrying P1::Tn5 maintained on LB agar plate containing 30 µg/ml of kanamycin was inoculated into 5 ml of P1 medium (LB supplemented with 0.01 M MgSO₄·7H₂O and 10 µg/ml thymine) and cultivated at 30°C overnight. One ml of this culture was transferred to 100 ml of the same medium in a 500 ml-Erlenmyer flask and grown to OD550 of approximately 0.09 at 30°C for 95 min. The resulting culture was cooled on ice for 10 min and centrifuged at 3500 rpm at 4°C for 20 min. The cells were suspended in 25 ml of P1 medium and the cell suspension was transferred to a 300 ml-flask and incubated at 42°C for 20 min without shaking. When lysis of the cell was observed by standing at 37°C for 90 min, the cells were lysed by adding 0.5 ml of chloroform. The mixture was then vortexed thoroughly, stood at room temperature for 10 min, and centrifuged at 10,000 rpm at 4°C for 15 min. After the supernatant was transferred into a sterile screw-top glass bottle, 0.5 ml of chloroform was added again and the lysate was stored at 4°C.

The recipient cell, L42-9 grown on No. 4 agar plate consisting of 0.5% glycerol, 0.5% yeast extract (Difco) and 0.5% MgSO₄·7H₂O was inoculated into a tube containing 5 ml of No. 4 medium and grown on a tube shaker at 30°C overnight. One ml of the culture was transferred to 30 ml of the same medium in a 500-Erlenmyer flask and cultured at 30°C for 3 hr. The culture was centrifuged for 15 min. The resulting pellet was suspended in 1.8 ml of 100 mM MC buffer consisting of 100 mM MgSO₄·7H₂O and 100 mM CaCl₂. 0.1 ml of the cell suspension was mixed with 0.1 ml of phage solution diluted 10-fold with 10 mM MC buffer in the tube and placed at 30°C for 60 min. After 0.8 ml of No. 4 medium was added, the tube was incubated at room temperature for 2 hr. The infected cell suspension of the volume of 0.2 ml was then spread on agar plates of No. 4 medium containing 100 µg/ml of kanamycin, and incubated at 27°C for 5 days.

### (ii) Screening of L-sorbose reductase-deficient mutants by product assay

Strains with Tn5 (Km^{r}) grown on No. 4-Km agar plate at 27°C for 4 days were suspended in 50 µl of 0.15M citrate-Na₂HPO₄ buffer (pH8.0) solution containing 4% L-sorbose per well of a 96 wells-microtiter plate and incubated at 30°C for 24 hr without shaking. D-Sorbitol converted from L-sorbose was analyzed by thin layer chromatography (TLC) using 1µl of the sample. The TLC analysis was carried out by using TLC plate of Kieselgel 60 F₂₅₄, Merck; solvent of ethyl acetate/isopropyl alcohol/acetic acid/H₂O = 10:6:3.5:3; and spray reagents of 0.5% KIO₄ solution and a tetrabase reagent prepared by mixing 2N-acetate saturated with tetrabase and 15% MnSO₄·4-6 H₂O (1:1 by volume).

By theTLC analysis, one mutant designated 26-9A was obtained as a candidate of L-sorbose reductase-deficient mutant. Strain 26-9A produced a faint amount of D-sorbitol from L-sorbose, while the other Tn5-mutants and the parent L42-9 produced significant amount of D-sorbitol.

### (iii) Determination of L-sorbose reductase activity

Cells grown on 8% sorbitol-No. 5 medium consisting of 8% D-sorbitol, 1.5% yeast extract (Oriental Yeast Co., Osaka, Japan), 0.25% MgSO₄·7H₂O, 0.05% glycerol and 1.5% CaCO₃ (production grade) at 30°C for 2 days were harvested by centrifugation and washed with 0.3% NaCl solution twice. The cell paste was suspended in 10 mM KH₂PO₄-K₂HPO₄ buffer (pH7.0), and passed through a French pressure cell press twice. After centrifugation to remove intact cells, the supernatant was centrifuged at 100,000 x g for 60 min. The resulting supernatant was collected as a source of L-sorbose reductase.

L-Sorbose reductase activity was determined by photometric assay in the presence of NADPH (T. Sugisawa et al., Agric. Biol. Chem., 55: 2043-2049, 1991). The reaction mixture contained 5 mg/ml of L-sorbose, 0.4 mg/ml of NADPH in 50 mM KH₂PO₄-K₂HPO₄ buffer (pH7.0) and 10 µl of enzyme solution. The change in absorption resulting from substrate-dependent oxidation of NADPH was followed at 340 nm with a Kontron spectrophotometer UVIKON 810. One unit of the reductase activity was defined as the amount of the enzyme catalyzing the formation of 1 µmole of NADP per min.

L-Sorbose reductase activities of strain 26-9A and its parent L42-9 were determined as described above to be less than 0.01 and 0.21 units/mg protein, respectively.

### (iv) Colony hybridization

Introduction of Tn5 fragment to the chromosome of the Strain 26-9A was confirmed by a colony blot hybridization with ³²P-labeled Col E1::Tn5 DNA as the probe.

### Example 2. Cloning and nucleotide sequencing of Tn5-inserted region

New Tn5-mutants were re-constructed by using pSUP202 (Ap^{r}Cm^{r}Tc^{r}'; SimonR. et al., BIO/TECHNOL., 1: 784-791, 1983) with the DNA fragment containing Tn5 to confirm that L-sorbose reductase deficiency of 26-9A was caused by the Tn5 insertion, not by different mutation simultaneously occurred on the different position of 26-9A DNA from the position of the Tn5 insertion. Southern-blot hybridization of various DNA fragments of strain 26-9A chromosome revealed that 13 kb EcoRV fragment containing a whole Tn5 had enough length (more than at least 1 kb) of DNA at both sides of the Tn5 insertion point for a double-crossing over recombination. The *Eco* RV fragment was cloned in pSUP202 to produce pSR02, which was then introduced into *G. melanogenus* IFO 3293 to give Km^{r}Cm^{s} strains, 3293EV-1 and 3293EV-9. Southern-blot hybridization analysis of the strains 3293EV-1 and 3293EV-9 revealed that both strains contained Tn5 without pSUP202 vector portion as strain 26-9A did (data not shown), indicating that homologous recombination by a double crossover was occurred. Deficiency of L-sorbose reductase activity in the strains 3293EV-1 and 3293EV-9 was examined by a product assay and a photometric enzyme assay as performed for strain 26-9A; new Tn5 mutants also produced undetectable L-sorbose and showed L-sorbose reductase activity below 0.01 unit/mg cytosol protein, while *G. melanogenus* IFO 3293 showed L-sorbose reductase activity of 0.20 units/mg cytosol protein. It should be concluded that the Tn5 insertion in 26-9A caused L-sorbose reductase deficiency.

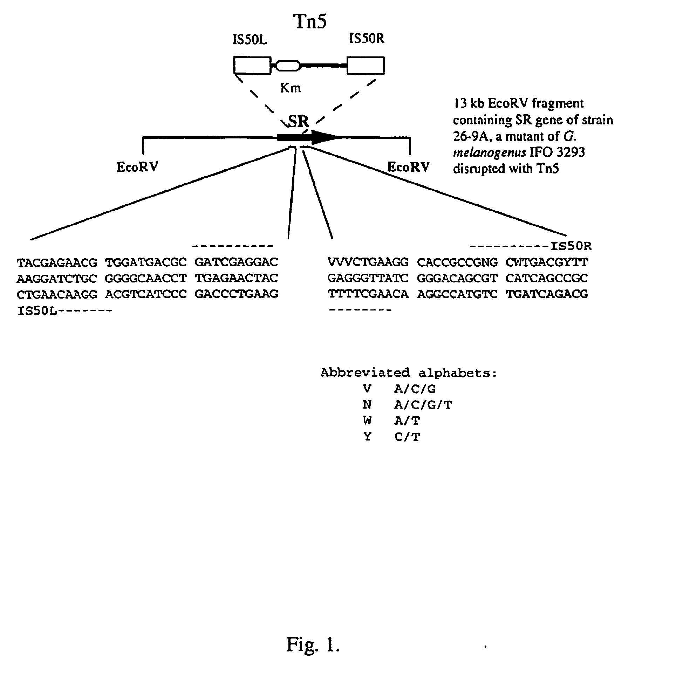

The nucleotide sequence of Tn5-inserted region on pSR02 was determined by the dideoxy chain termination method (Sanger F. et al., Proc. Natl. Acad. Sci. USA., 74: 5463-5467, 1977) (Fig. 1). Analysis of the Tn5-inserted region by the homology search with BLASTP program (Lipman D. J. et al., J. Mol. Biol. 215: 403-410, 1990) revealed that the region encodes a polypeptide having a homology with polypeptides belonging to mannitol dehydrogenase (MDH) family. One of the member, mannitol 2-dehydrogenase (EC 1.1.1.67) of *Rhodobacter sphaeroides* (Schneider K.-H. et al, J. Gen. Microbiol., 1993) catalyzes the NAD-dependent oxidation of mannitol into fructose. L-Sorbose reductase of *Gluconobacter* catalyzes not only the reduction of L-sorbose and D-fructose to produce D-sorbitol and D-mannitol in the presence of NADPH but also the oxidation of D-sorbitol and D-mannitol to produce L-sorbose and D-fructose in the presence of NADP (Sugisawa T. et al., ibid). The homology analysis indicated that polypeptide encoded by Tn5-disrupted gene of strain 26-9A is L-sorbose reductase gene itself, not its regulatory gene.

### Example 3. PCR cloning.

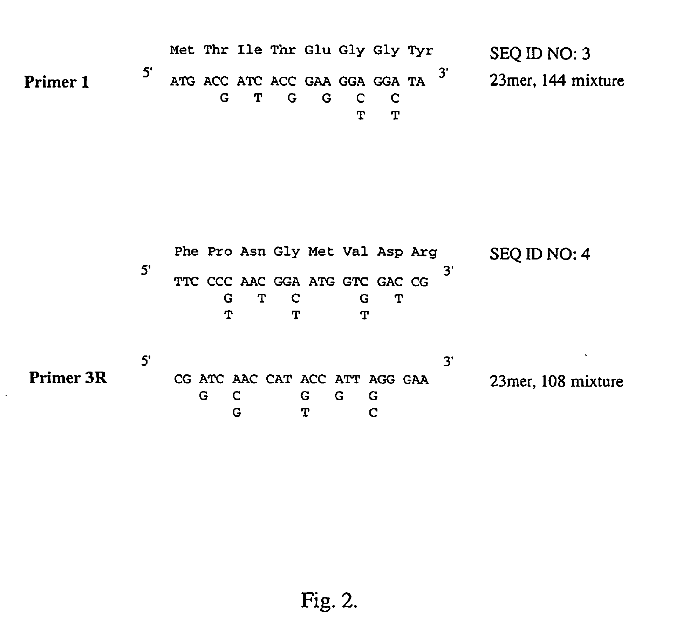

Partial L-sorbose reeducate gene of *G. suboxydans* IFO 3291 was cloned by PCR amplification with a set of primers synthesized according to the amino acid sequences (SEQ ID NO: 3 and SEQ ID NO: 4) shown in Fig. 2; the degenerate primers were synthesized in consideration of the codon usage of *Gluconobacter*. The PCR gave about 300 bp product. Through Southern- and colony-hybridization analyses using this PCR-amplified fragment as a probe, complete L-sorbose reductase gene of *G. suboxydans* IFO 3291 was obtained in an 8.0 kb EcoRV fragment.

### Example 4. Determination of nucleotide sequence of L-sorbose reductase gene from G. suboxydans IFO 3291.

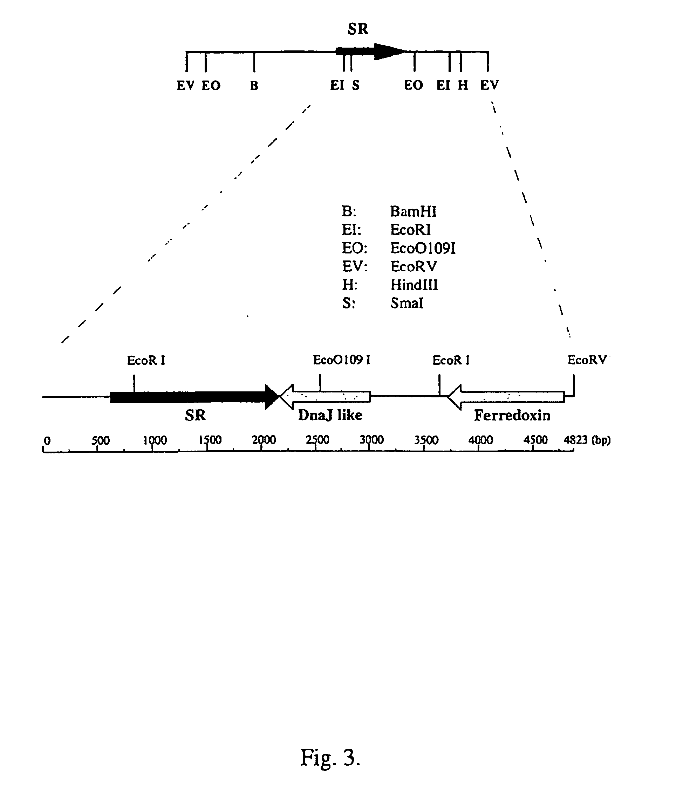

Complete nucleotide sequence of the L-sorbose reductase gene was determined with 8.0 kb of *Eco* RV fragment containing the L-sorbose reductase gene of *G. suboxydans* IFO 3291. The nucleotide sequence and deduced amino acid sequence are shown in SEQ ID NO: 1 and SEQ ID NO: 2. The restriction map of the 8.0 kb *Eco* RV is shown in Fig. 3. SR gene and two open reading frames, which were found to be downstream of the L-sorbose reductase gene and encode polypeptides having homologies with DnaJ-like protein and ferredoxin, are located in the opposite direction. Upstream of the L-sorbose reductase gene, no relevant ORFs which possibly make an operon with the L-sorbose reductase gene, were found. Therefore, disruption of the L-sorbose reductase gene was considered not to affect the expression of its neighboring genes.

### Example 5. Construction of L-sorbose reductase gene disruptant from G. suboxydans IFO 3291.

As can be taken form Fig. 4, the plasmid pUC4K, pSR03-1, and pSUP202 are the starting material plasmids. pUC4K is a source of Km resistant gene cassette and is available from Pharmacia (Uppsala, Sweden; Code No. 27-4958-01). The plasmid pSR03-1 is a derivative of the commercially available vector, pBluescript II SK (Stratagene CA, usa; Catalog No. 212205 and 212206) carrying *Eco*RV fragment obtained in Example 3. The vector pSUP202 is a derivative of pBR325 carrying a fragment containing a mob site, and as it is well known, pBR325 is a derivative of pBR 322. Although pBR itself dose not seem to be comercially available, an alternative material, e.g. pBR322 (ATCC 31344), pACYC177 (ATCC 37031) or pACYC184 (ATCC 37033) is believed to be readily applicable for the person skilled in the art, as described as pSUP series by Simon R. et al. (1983, Bio/Technology 1: 784-791). Construction of a mob site-containing plasmid is also be reported by Simon R. et al. (ibid).

Figure 4 shows the scheme for the construction of a L-sorbose reductase gene targeting vector, pSUP202-SR::Km. *Eco* RV fragment of 8.0 kb was cloned in the *Eco* RI site of pBluescript II SK vector (Alting-Mees M. A. et al., Methods in enzymology 216: 483-95, Academic Press, London, 1992) to produce pSR03-1. A kanamycin-resistant-gene cassette (Km cassette) was inserted into an *Eco* RI site in the cloned L-sorbose reductase gene to obtain pSR04-1. This L-sorbose reductase gene disrupted with Km cassette was subcloned in a suicide vector pSUP202. The resulting pSR05-2, pSUP202-SR::Km (Km^{r}), was then introduced into *G. suboxydans* IFO3291 to obtain L-sorbose reductase-null mutant (Km^{r}). The aimed gene disruption was finally confirmed by Southern-hybridization analysis. A schematic mechanism for the disruption is illustrated in Fig. 5. A L-sorbose reductase deficient mutant SR3 was thus obtained.
L-sorbose reductase-gene-targeted mutant, SR3, and *G. suboxydans* IFO 3291 showed L-sorbose reductase activity of below 0.02 and 0.681 units/mg protein, respectively.

### Example 6. Fermentation profile of strain SR3 in 8% sorbitol-No. 5 medium

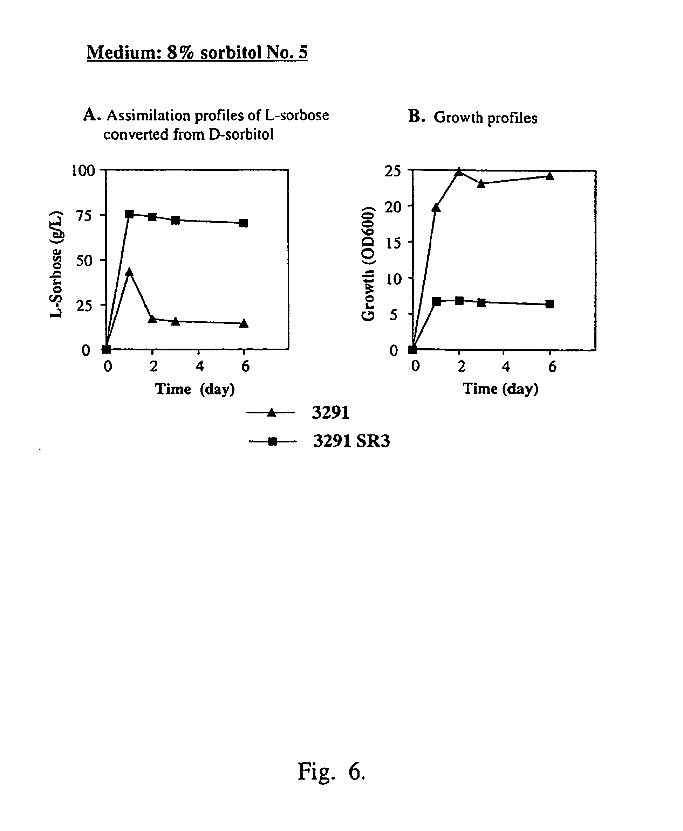

Growth and L-sorbose-assimilation profiles of strain SR3 and *G. suboxydans* IFO 3291 were evaluated with 8% sorbitol-No.5 medium in 500 ml Erlenmeyer flask (Fig. 6). Strain SR3 and *G. suboxydans* IFO 3291 could convert 80 g/L D-sorbitol to L-sorbose within 24 hr (data not shown). *G. suboxydans* IFO3291 assimilated most of the converted L-sorbose within 48 hr. On the other hand, strain SR3 hardly utilized L-sorbose until the 3rd day; the remaining L-sorbose was more than 70 g/L. Strain SR3 and *G. suboxydans* IFO 3291 showed OD600 (cell growth) of 6.4 and 24 after 6 days, respectively.

### Example 7. Fermentation profile of strain SR3 in 2% sorbitol-SCM medium

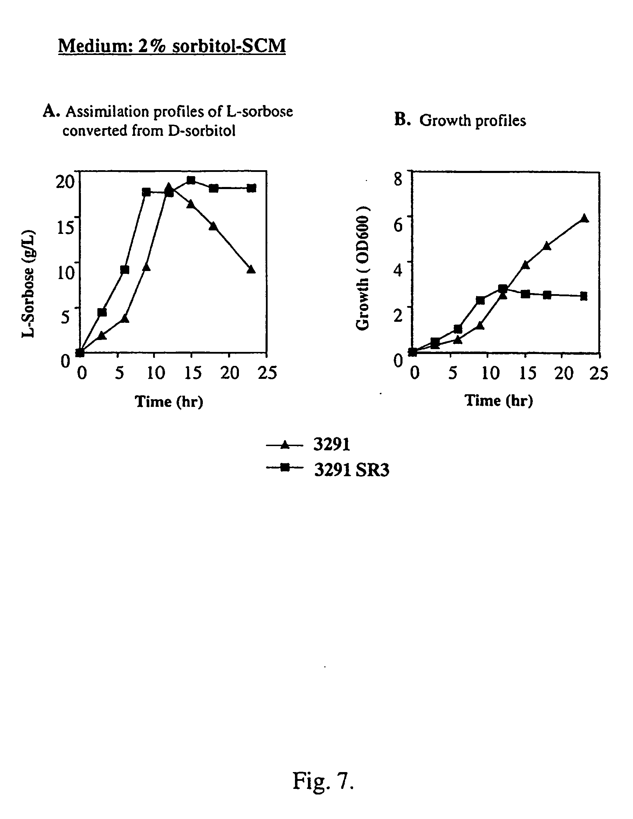

Growth and L-sorbose-assimilation profiles of strain SR3 and *G. suboxydans* IFO 3291 were evaluated with 2% sorbitol-SCM medium in 500 ml Erlenmeyer flask (Fig. 7). Strain SR3 and *G. suboxydans* IFO 3291 could convert 20 g/L D-sorbitol to L-sorbose within 12 hr. *G. suboxydans* IFO3291 assimilated half of the converted L-sorbose in 23 hr. On the other hand, strain SR3 hardly utilized L-sorbose. Strain SR3 and *G. suboxydans* IFO 3291 showed OD600 (cell growth) of 2.5 and 5.9 after 23 hr, respectively.

### Sequence Listing

### INFORMATION FOR SEQ ID NO: 1:

(i) SEQUENCE CHARACTERISTICS:
   (A) LENGTH: 1458 base pairs
   (B) TYPE: nucleic acid
   (C) STRANDEDNESS: double
   (D) TOPOLOGY: linear
(ii) MOLECULE TYPE: DNA (genomic)
(iii) ORIGINAL SOURCE:
   **ORGANISM:** *Gluconobacter suboxydans*
   STRAIN: IFO 3291
(iv) FEATURE:
   FEATURE KEY: CDS
   POSITION: 1..1458
   SEQUENCING METHOD: E

### INFORMATION FOR SEQ ID NO: 2:

(i) SEQUENCE CHARACTERISTICS:
   (A) LENGTH: 485 residues
   (B) TYPE: amino acid
   (C) TOPOLOGY: linear
(ii) MOLECULE TYPE: protein
(iii) ORIGINAL SOURCE:
   ORGANISM: *Gluconobacter suboxydans*
   STRAIN: IFO 3291
(iv) FEATURE:
   FEATURE KEY: mat peptide
   POSITION: 1..485
   SEQUENCING METHOD: E

### INFORMATION FOR SEQ ID NO: 3:

(i) SEQUENCE CHARACTERISTICS:
   (A) LENGTH: residues
   (B) TYPE: amino acid
   (C) TOPOLOGY: linear
(ii) MOLECULE TYPE: peptide
(iii) ORIGINAL SOURCE:
   ORGANISM: *Gluconobacter melanogenus*
   STRAIN: IFO 3293
(iv) FEATURE:
   FEATURE KEY:peptide
   SEQUENCING METHOD: E

### INFORMATION FOR SEQ NO: 4:

(i) SEQUENCE CHARACTERISTICS:
   (A) LENGTH: 8 residues
   (B) TYPE: amino acid
   (C) TOPOLOGY: linear
(ii) MOLECULE TYPE: peptide
(iii) ORIGINAL SOURCE:
   ORGANISM: *Gluconobacter melanogenus*
   STRAIN: IFO 3293
(iv) FEATURE:
   FEATURE KEY: peptide
   SEQUENCING METHOD : E

## Claims

1. A genetically engineered microorganism derived from a microorganism belonging to the genus *Gluconobacter* or *Acetobacter* comprising a gene encoding a protein having L-sorbose reductase activity, said gene having a stably integrated mutation, wherein said genetically engineered microorganism is deficient in the conversion of L-sorbose to D-sorbitol and wherein the L-sorbosereductase activity within said genetically engineered microorganism is less than 10% of the amount of the activity of the wild-type microorganism.

2. The genetically engineered microorganism according to claim 1, in which the gene thereof carries at least one mutation with the aid of disruption, addition, insertion, deletion and/or substitution within a region required for the formation of active L-sorbose reductase.

3. The genetically engineered microorganism according to claim 2, in which the disruption contains at least one interfering DNA fragment selected from the group consisting of a transposon, an antibiotic resistant gene cassette and DNA sequences which prevent the host microorganism from formation of active L-sorbose reductase.

4. The genetically engineered microorganism according to claims 2 or 3, in which the said region required for the formation of active L-sorbose reductase lies in the DNA sequence selected from the group consisting of a structural gene encoding L-sorbose reductase and the expression control sequences therefor.

5. The genetically engineered microorganism according to any one of claims 1 to 4 wherein the gene encoding a protein having L-sorbose reductase activity is selected from the group consisting of SEQ ID NO: 1, a fragment of SEQ NO: 1 encoding a polypeptide having L-sorbose reductase activity and a polynucleotide encoding SEQ ID NO: 2.

6. Use of an L-sorbose reductase gene for the production of a genetically engineered microorganism belonging to the genus *Gluconobacter* or *Acetobacter* according to any one of claims 1 to 5 wherein said gene is **characterized in that** it encodes the amino acid sequence of L-sorbose reductase described in SEQ ID NO: 2 or its functional equivalent containing insertion, deletion, addition and/or substitution of one or more amino acid(s) in said SEQ ID NO: 2.

7. A method for producing L-sorbose by the fermentation of a microorganism in an appropriate medium, which comprises use of the said genetically engineered microorganism defined in any one of claims 1 to 5.

8. A method for producing vitamin C comprising a fermentation step for the production of L-sorbose, which is **characterized in that** the process comprises use of a genetically engineered microorganism defined in any one of claims 1 to 5.

9. A process for the production of a genetically engineered microorganism according to claim 1 comprising:
(a) providing a microorganism of the genus *Gluconobacter* or *Acteobacter* comprising a gene encoding a protein having L-sorbose reductase activity, and
(b) introducing a mutation in said polynucleotide to result in a biological activity for reducing L-sorbose which is less than 10% of the amount of the activity of the wild-type microorganism.

10. A process for the production of a genetically engineered microorganism comprising the steps of:
(a) providing a microorganism of the genus *Gluconobacter* or *Acteobacter* comprising a gene encoding a protein having L-sorbose reductase activity,
(b) genetically engineering said microorganism by introducing a stable mutation into said gene,
(c) determining the L-sorbose reductase activity of the microorganism comprising the mutated gene obtained in step (b),
(d) selecting a microorganism based on step (c) in which L-sorbose reductase activity is less than 10% of the wild-type microorganism.

11. A process according to claim 9 or 10 wherein the microorganism is genetically engineered by gene disruption, addition, insertion, deletion and/or substitution within a region required for the formation of active L-sorbose reductase.

12. A process according to any one of claims 9 to I 1 wherein the gene coding for L-sorbose reductase is selected from the group consisting of SEQ ID NO: 1, a fragment of SEQ ID NO: 1 encoding a polypeptide having L-sorbose reductase activity and a polynucleotide encoding SEQ ID NO: 2.

13. A process for the production of L-sorbose or vitamin C comprising a fermentation step for the production of L-sorbose in an appropriate medium with a microorganism as produced by a process according to any one of claims 9 to 12.

## Patentansprüche

1. Genetisch veränderter Mikroorganismus, der von einem Mikroorganismus der Gattung *Gluconobacter* oder *Acetobacter* abstammt, und der ein Gen enthält, das für ein Protein mit L-Sorbosereduktaseaktivität codiert, wobei dieses Gen eine stabil integrierte Mutation aufweist, wobei besagter genetisch veränderte Mikroorganismus bezüglich der Umwandlung von L-Sorbose in D-Sorbitol defizient ist und wobei die L-Sorbosereduktaseaktivität in diesem genetisch veränderten Mikroorganismus weniger als 10% der Menge der Aktivität des Wildtyp-Mikroorganismus beträgt.

2. Der genetisch veränderte Mikroorganismus nach Anspruch 1, wobei dessen Gen mindestens eine Mutation mittels Disruption, Addition, Insertion, Deletion und/oder Substitution innerhalb einer Region, die für die Bildung einer aktiven L-Sorbosereduktase erforderlich ist, trägt.

3. Der genetisch veränderte Mikroorganismus nach Anspruch 2, wobei die Disruption mindestens ein interferierendes DNA-Fragment ausgewählt aus der Gruppe bestehend aus einem Transposon, einer Antibiotikaresistenz-Genkassette und DNA-Sequenzen, die den Wirtsorganismus an der Bildung einer aktiven L-Sorbosereduktase hindern, enthält.

4. Der genetisch veränderte Mikroorganismus nach Anspruch 2 oder 3, wobei besagte für die Bildung einer aktiven L-Sorbosereduktase erforderliche Region in der DNA-Sequenz ausgewählt aus der Gruppe bestehend aus einem Strukturgen, das für L-Sorbosereduktase codiert, und seinen Expressionskontrollsequenzen, liegt.

5. Der genetisch veränderte Mikroorganismus nach einem der Ansprüche 1 bis 4, wobei das Gen, das für ein Protein mit L-Sorbosereduktaseaktivität codiert, ausgewählt ist aus der Gruppe bestehend aus SEQ ID NO:1, einem Fragment von SEQ ID NO:1, das für ein Polypeptid mit L-Sorbosereduktaseaktivität codiert, und einem Polynukleotid, das für SEQ ID NO:2 codiert.

6. Verwendung eines L-Sorbosereduktasegens für die Erzeugung eines genetisch veränderten Mikroorganismus der Gattung *Gluconobacter* oder *Acetobacter* nach einem der Ansprüche 1 bis 5, wobei besagtes Gen **dadurch gekennzeichnet ist, daß** es für die Aminosäuresequenz der in SEQ ID NO:2 beschriebenen L-Sorbosereduktase oder ihr funktionelles Äquivalent, das eine Insertion, Deletion, Addition und/oder Substitution von einer oder mehreren Aminosäure(n) in dieser SEQ ID NO:2 enthält, codiert.

7. Verfahren zur Herstellung von L-Sorbose durch Fermentation eines Mikroorganismus in einem entsprechenden Medium, umfassend die Verwendung von besagtem, in Ansprüchen 1 bis 5 definierten, genetisch verändertem Mikroorganismus.

8. Verfahren zur Herstellung von Vitamin C, umfassend einen Fermentationsschritt für die Herstellung von L-Sorbose, **dadurch gekennzeichnet, daß** das Verfahren die Verwendung eines, in ein in einem der Ansprüche 1 bis 5 definierten, genetisch veränderten Mikroorganismus umfasst.

9. Verfahren zur Herstellung eines genetisch veränderten Mikroorganismus nach Anspruch 1, umfassend:
(a) Bereitstellen eines Miroorganismus der Gattung *Gluconobacter* oder *Acetobacter,* der ein Gen enthält, das für ein Protein mit L-Sorbosereduktaseaktivität codiert, und
(b) Einführen einer Mutation in dieses Polynukleotid, die zu einer biologischen Aktivität zur Reduktion von L-Sorbose führt, die weniger als 10% der Aktivitätsmenge des Wildtyp-Mikroorganismus beträgt.

10. Verfahren zur Herstellung eines genetisch veränderten Mikroorganismus, umfassend die folgenden Schritte:
(a) Bereitstellen eines Miroorganismus der Gattung *Gluconobacter* oder *Acetobacter*, der ein Gen enthält, das für ein Protein mit L-Sorbosereduktaseaktivität codiert,
(b) genetisches Verändern dieses Mikroorganismus durch Einführen einer stabilen Mutation in dieses Gen,
(c) Bestimmen der L-Sorbosereduktaseaktivität des Mikroorganismus, der das in Schritt (b) erhaltene mutierte Gen enthält,
(d) Selektieren eines Mikroorganismus basierend auf Schritt (c), in dem die L-Sorbosereduktaseaktivität weniger als 10% des Wildtyp-Organismus beträgt.

11. Verfahren nach Anspruch 9 oder 10, wobei der Mikroorganismus durch Gendisruption, -addition, -insertion, -deletion und/oder -substitution innerhalb einer Region, die für die Bildung einer aktiven L-Sorbosereduktase erforderlich ist, genetisch verändert wird.

12. Verfahren nach einem der Ansprüche 9 bis 11, wobei das Gen, das für die L-Sorbosereduktase codiert, ausgewählt ist aus der Gruppe bestehend aus SEQ ID NO:1, einem Fragment von SEQ ID NO:1, das für ein Polypeptid mit L-Sorbosereduktaseaktivität codiert, und einem Polynukleotid, das für SEQ ID NO:2 codiert.

13. Verfahren zur Herstellung von L-Sorbose oder Vitamin C, umfassend einen Fermentationsschritt für die Herstellung von L-Sorbose in einem entsprechenden Medium mit einem Mikroorganismus, der mit einem Verfahren nach einem der Ansprüche 9 bis 12 hergestellt wird.

## Revendications

1. Microorganisme génétiquement modifié dérivé d'un microorganisme appartenant au genre *Gluconobacter* ou *Acetobacter* comprenant un gène codant pour une protéine ayant une activité L-sorbose réductase, ledit gène comprenant une mutation intégrée de façon stable, ledit microorganisme génétiquement modifié étant déficient en ce qui concerne la conversion du L-sorbose en D-sorbitol et l'activité L-sorbose réductase dans ledit microorganisme génétiquement modifié étant inférieure à 10 % du niveau de l'activité du microorganisme de type sauvage.

2. Le microorganisme génétiquement modifié selon la revendication 1, dans lequel le gène porte au moins une mutation obtenue par disruption, addition, insertion, délétion et/ou substitution dans une région requise pour la formation d'une L-sorbose réductase active.

3. Le microorganisme génétiquement modifié selon la revendication 2, dans lequel la disruption contient au moins un fragment d'ADN interférant choisi dans le groupe constitué par un transposon, une cassette de gène de résistance aux antibiotiques et des séquences d'ADN qui empêchent le microorganisme hôte de former une L-sorbose réductase active.

4. Le microorganisme génétiquement modifié selon la revendication 2 ou 3, dans lequel ladite région requise pour la formation d'une L-sorbose réductase actiye se situe dans la séquence d'ADN choisie dans le groupe constitué par un gène structural codant pour la L-sorbose réductase et les séquences de contrôle de l'expression de celui-ci.

5. Le microorganisme génétiquement modifié selon l'une quelconque des revendications 1 à 4, le gêne codant pour une protéine ayant une activité L-sorbose réductase étant choisi dans le groupe constitué par SEQ ID NO : 1, un fragment de SEQ ID NO : 1 codant pour un polypeptide ayant une activité L-sorbose réductase et un polynucléotide codant pour SEQ ID NO : 2.

6. Utilisation d'un gène de L-sorbose réductase pour la production d'un microorganisme génétiquement modifié appartenant au genre *Gluconobacter* ou *Acetobacter* selon l'une quelconque des revendications 1 à 5, ledit gène étant **caractérisé en ce qu'**il code pour la séquence d'acides aminés de la L-sorbose réductase décrite par SEQ ID NO : 2 ou de son équivalent fonctionnel contenant une insertion, une délétion une addition et/ou une substitution d'un ou de plusieurs acides aminés dans ladite SEQ ID NO : 2.

7. Méthode de production de L-sorbose par la fermentation d'un microorganisme dans un milieu approprié, qui comprend l'utilisation dudit microorganisme génétiquement modifié défini dans l'une quelconque des revendications 1 à 5.

8. Méthode de production de vitamine C comprenant une étape de fermentation pour la production de L-sorbose, qui est **caractérisée en ce que** le procédé comprend l'utilisation d'un microorganisme génétiquement modifié défini dans l'une quelconque des revendications 1 à 5.

9. Procédé de production d'un microorganisme génétiquement modifié selon la revendication 1, comprenant :
(a) obtenir un microorganisme du genre *Gluconobacter* ou *Acetobacter* comprenant un gène codant pour une protéine ayant une activité L-sorbose réductase, et
(b) introduire une mutation dans ledit polynucléotide pour aboutir à une activité biologique de réduction du L-sorbose qui est inférieure à 10 % du niveau de l'activité du microorganisme de type sauvage.

10. Procédé de production d'un microorganisme génétiquement modifié comprenant les étapes consistant à :
(a) obtenir un microorganisme du genre *Gluconobacter* ou *Acetobacter* comprenant un gène codant pour une protéine ayant une activité L-sorbose réductase,
(b) modifier génétiquement ledit microorganisme en introduisant une mutation stable dans ledit gène,
(c) déterminer l'activité L-sorbose réductase du microorganisme comprenant le gène muté obtenu dans l'étape (b),
(d) sélectionner un microorganisme en se basant sur l'étape (c), dans lequel l'activité L-sorbose réductase est inférieure à 10 % de celle du microorganisme de type sauvage.

11. Procédé selon la revendication 9 ou 10, selon lequel le microorganisme est modifié génétiquement par disruption génique, addition, insertion, délétion et/ou substitution dans une région requise pour la formation d'une L-sorbose réductase active.

12. Procédé selon l'une quelconque des revendications 9 à 11, selon lequel le gène codant pour la L-sorbose réductase est choisi dans le groupe constitué par SEQ ID NO : 1, un fragment de SEQ ID NO : 1 codant pour un polypeptide ayant une activité L-sorbose réductase et un polynucléotide codant pour SEQ ID NO : 2.

13. Procédé de production de L-sorbose ou de vitamine C comprenant une étape de fermentation pour la production de L-sorbose dans un milieu approprié avec un microorganisme tel que produit par un procédé selon l'une quelconque des revendications 9 à 12.
